# NOUVEAU FASCICULE DE BREVET EUROPEEN

(11) **EP 3 151 673 B2**
(45) Date de publication et mention de la décision concernant l'opposition: **14.12.2022**
(45) Mention de la délivrance du brevet: 19.02.2020
(21) Numéro de dépôt: 15727959.7
(22) Date de dépôt: 05.06.2015
(51) Int. Cl.: A01N 63/04

(54) **MÉTHODE ET COMPOSITION POUR AMÉLIORER LA PRODUCTIVITÉ DE PLANTES NON LÉGUMINEUSES**
VERFAHREN UND ZUSAMMENSETZUNG ZUR VERBESSERUNG DER PRODUKTIVITÄT VON NICHTHÜLSENFRÜCHTEN
METHOD AND COMPOSITION FOR IMPROVING THE PRODUCTIVITY OF NON-LEGUMINOUS PLANTS

(30) Priorité: 06.06.2014 FR 1455181
(43) Date de publication de la demande: 12.04.2017
(73) Titulaire: Lesaffre et Compagnie, 75001 Paris (FR)
(72) Inventeur: MANCEAU, Florian, F-49000 Angers (FR); MARTIN, Antoine, F-49070 Beaucouze (FR); PAJOT, Emmanuel, F-49123 Champtocé-sur-Loire (FR); PUJOS, Philippe, F-33170 Gradignan (FR); REVEILLAUD, Maud-Cécile, F-49800 Trélazé (FR)
(74) Mandataire: Bandpay & Greuter
(86) Numéro de dépôt international: PCT/EP2015/062554
(87) Numéro de publication internationale: WO 2015/185717

(56) Documents cités:
- WO-A1-2014/160827
- FR-A1- 2 873 688
- FR-A1- 2 901 271
- US-A- 4 434 231
- US-A1- 2007 110 725
- US-A1- 2010 024 076
- KOBAYASHI MICHIHARU ET AL: "Effect of yeast extracts on higher plants", PLANT AND SOIL, KLUWER ACADEMIC PUBLISHERS, NL, vol. 57, no. 1, 1 janvier 1980 (1980-01-01), pages 41-47, XP009182370, ISSN: 0032-079X
- M S HANAFY ET AL: "Effect of Some Natural Extracts on Growth and Chemical Constituents of Schefflera arboricola Plants", JOURNAL OF HORTICULTURAL SCIENCE & ORNAMENTAL PLANTS, vol. 4, 1 janvier 2012 (2012-01-01), pages 26-33, XP055167223,
- I. SAMPEDRO ET AL: "Improvement by soil yeasts of arbuscular mycorrhizal symbiosis of soybean (Glycine max) colonized by Glomus mosseae", MYCORRHIZA, vol. 14, no. 4, 18 décembre 2003 (2003-12-18), pages 229-234, XP055168237, ISSN: 0940-6360, DOI: 10.1007/s00572-003-0285-y
- ERIK VERBRUGGEN ET AL: "Mycorrhizal fungal establishment in agricultural soils: factors determining inoculation success", NEW PHYTOLOGIST, vol. 197, no. 4, 26 septembre 2012 (2012-09-26), pages 1104-1109, XP055167280, ISSN: 0028-646X, DOI: 10.1111/j.1469-8137.2012.04348.x
- SMITH et al.: "Roles of Arbuscular Mycorrhizas in Plant Nutrition and Growth: New Paradigms from Cellular to Ecosystem Scales", Annu. Rev. Plant Biol, vol. 62, 2011, pages 227-250, DOI: 10.1146/annurev-arplant-042110-103846
- ANGELARD, C. et al.: "Segregation in a mycorrhizal fungus alters rice growth and symbiosis-specific gene transcription", Curr Biol, vol. 20, 2010, pages 1216-1220,
- WANG et al.: "Phylogenetic distribution and evolution of mycorrhizas in land plants", Mycorrhiza, vol. 16, 2006, pages 299-363, DOI: 10.1007/s00572-005-0033-6
- BRUNDRETT, M.: "Mycorrhizal associations and other means of nutrition of vascular plants: understanding the global diversity of host plants by resolving conflicting information and developing reliable means of diagnosis", Plant Soil, vol. 320, 2009, pages 37-77,
- SMITH, S. E. et al.: "Functional diversity in arbuscular mycorrhizal (AM) symbioses: the contribution of the mycorrhizal P uptake pathway is not correlated with mycorrhizal responses in growth or total P uptake", New Physiologist, vol. 162, 2004, pages 511-524,
- GRACE, E. J. et al.: "Arbuscular mycorrhizal inhibition of growth in barley cannot be attributed to extent of colonization, fungal phosphorus uptake or effects on expression of plant phosphate transporter genes", New Physiologist, vol. 181, 2009, pages 938-949,
- SMITH, S. E. et al.: "Roles of arbuscular mycorrhizas in plant phosphorous nutrition: interactions between pathways of phosphorous uptake in arbuscular mycorrhizal roots have important implications for understanding and manipulating plant phosphorus acquisition", Plant Physiol., vol. 156, 2011, pages 1050-1057,
- Difco? Yeast Extract, ultra-filtered (UF) product datasheet,
- ALEKLETT et al.: "Effects of organic amendments with various nitrogen levels on arbuscular mycorrhizal fungal growth", Applied Soil Ecology, vol. 60, 2012, pages 71-76, DOI: 10.1016/j.apsoil.2012.03.007
- "Yeast Extract Product 1.03753 datasheet", Merck MICROBIOLOGY MANUAL,
- , Retrieved from the Internet: URL:http://amf-phylogeny.com/webpage
- PARNISKE: "Arbuscular mycorrhiza: the mother of plant root endosymbioses", Nature Reviews Microbiology, vol. 6, 2008, pages 763-775,
- COSME et al.: "Non Mycorrhizal Plants: The Exceptions that Prove the Rule", Trends in Plant Science, vol. 23, 2018, pages 577-587,
- Mcgonigle T P, Et Al: "A new method which gives an objective measure of colonization of roots by vesicular-arbuscular mycorrhizal fungi", New Phytol, vol. 115, 1 January 1990 (1990-01-01), pages 495-502,
- Olsson P A, Et Al: "Elemental composition in vesicles of an arbuscular mycorrhizal fungus, as revealed by PIXE analysis", Fungal Biology, vol. 115, 1 January 2011 (2011-01-01), pages 643-648,
- Vierheilig H, Et Al: "Ink and vinegar, a simple staining technique for arbuscular-mycorrhizal fungi", Applied and Environmental Microbiology, vol. 64, no. 12, 1 December 1998 (1998-12-01), pages 5004-5007,

## Description

### DOMAINE DE L'INVENTION

La présente invention concerne le domaine de la culture des plantes, en particulier celui de la culture de plantes non-légumineuses. Plus particulièrement, la présente invention concerne une méthode d'amélioration de la productivité de plantes non légumineuses mettant en œuvre au moins une mycorhize et au moins un extrait de levure, ainsi qu'une composition comprenant une association de mycorhize(s) et d'extrait(s) de levure, et optionnellement un substrat.

### ÉTAT DE LA TECHNIQUE

L'agriculture cherche constamment à améliorer la productivité des cultures, pour améliorer la compétitivité des acteurs économiques et satisfaire les besoins croissants des marchés. Cette amélioration passe d'une part par l'accroissement des rendements, et l'amélioration des caractéristiques qualitatives, et d'autre part par la réduction des volumes et des coûts des intrants utilisés pour produire une unité de produit. De plus, dans le but de protéger l'environnement, et d'instaurer des pratiques agricoles durables, de nombreuses recherches visent à augmenter la productivité sans augmenter les apports d'intrants chimiques, voire en les réduisant. Un moyen d'atteindre ces objectifs est d'utiliser des microorganismes symbiotiques aidant les racines de la plante à utiliser au mieux les ressources disponibles. La méthode la plus courante pour ce faire est d'accroître la population de ces microorganismes par des apports de souches sélectionnées ou de souches prélevées localement et multipliées industriellement. Ces apports d'inoculum se font à proximité des racines, par exemple par apport au sol ou incorporation à un substrat, ou en traitement de semences. On utilise ainsi des bactéries *Rhizobium sp,* qui permettent le transfert de l'azote atmosphérique vers les plantes, ou des champignons mycorhiziens, qui s'associent aux racines des plantes et leur apportent des minéraux et de l'eau.

En particulier, Verbruggen E et al. (New Phytologist Volume 197, Issue 4, pages 1104-1109, March 2013) décrit que l'utilisation de mycorhizes améliore la productivité des plantes. De plus, Sampedro et al. (Mycorrhiza. 2004 Aug;14(4):229-34) décrit un effet favorable de levures vivantes sur les longueurs de spores des mycorhizes.

L'inoculation des racines par des champignons mycorhiziens permet d'accroître la productivité de la plante. Cependant les effets obtenus sont parfois insuffisants, ce qui freine le développement de cette technique, et il est nécessaire d'améliorer la méthode afin qu'elle réponde mieux aux exigences de ses utilisateurs.

Dans ce but, un procédé a été développé visant à améliorer la production de biomasse de plantes non légumineuses par l'application au sol d'inocula de mycorhizes et de levures inactives (FR2901271).

FR2901271 montre que les levures inactives exercent une action bénéfique sur les cultures de non légumineuses en application au sol avec des mycorhizes. Ce résultat s'est avéré surprenant, selon le Titulaire de cette demande de brevet, car d'après lui, il était connu que l'utilisation de levures mortes (les levures inactives sont des levures mortes) ou de fractions de levure n'avaient pas d'effet sur la mycorhization des plantes non légumineuses.

Ainsi, pour l'homme du métier, selon cet enseignement, les fractions de levure (par exemple des parois de levure ou des extraits de levure) n'ont pas d'effet sur la mycorhization des plantes non légumineuses. A fortiori, les extraits de levure (qui sont des fractions de levures) n'ont pas d'effet sur la mycorhization des plantes non légumineuses.

Par ailleurs, Kobayashi et al. (Plant and Soil 1980, Volume 57, Issue 1, pp 41-47) décrit une composition comprenant un extrait de levure *Saccharomyces Cerevisiae* extraite par autolyse pour améliorer la croissance d'une plante (« *vineless pea* »), étant entendu que les pois sont généralement des légumineuses.

Hanafy et al. (Plants Journal of Horticultural Science & Ornamental Plants 4 (1): 26-33, 2012) décrit l'effet d'un extrait de levure *Saccharomyces Cerevisiae* sur la croissance d'un arbre tropical Schefflera. FR2873688 décrit l'utilisation de levures actives ou inactives pour améliorer la nutrition des tomates ou de l'herbe.

Toutefois, aucun de ces documents ne mentionnent ni ne suggèrent que les extraits de levure pourraient présenter des effets sur la mycorhization lorsqu'ils sont appliqués avec des mycorhizes. Par ailleurs, ces documents ne suggèrent pas que l'utilisation combinée d'extraits de levure ou de mycorhize peut permettre d'observer un effet synergique, ainsi que montré dans un exemple de la présente demande.

Allant à l'encontre du préjugé de l'homme du métier, la Demanderesse a testé des associations d'extrait de levure et de mycorhize et ont constaté, de manière surprenante car totalement contraire à l'enseignement antérieur, qu'elles avaient un effet bénéfique, aussi bien en tant que telles, que mélangées à un substrat, et que l'utilisation d'un tel substrat enrichi d'extrait de levure et de mycorhizes permettait d'obtenir au moins un des résultats surprenants suivants :
- une amélioration de la mycorhization de cultures de non légumineuses, moyen permettant l'amélioration des autres paramètres,
- une amélioration de l'enracinement de cultures de non légumineuses,
- une amélioration de la croissance de cultures de non légumineuses,
- une amélioration de la floraison (quantité et précocité) de cultures de non légumineuses,
- une amélioration de la biomasse fraîche de cultures de non légumineuses,
- une amélioration de la biomasse sèche de cultures de non légumineuses,
- une amélioration des rendements de cultures de non légumineuses,
- une amélioration de la nutrition minérale de non légumineuses,
- une amélioration de la nutrition hydrique et de la résistance au stress hydrique.

Fondée sur l'incroyable efficacité de ces résultats, la présente invention a pour objet une méthode pour améliorer au moins l'un des paramètres ci-dessus mettant en œuvre une composition comprenant au moins une mycorhize et au moins un extrait de levure.

### DÉFINITIONS

Dans la présente invention, les termes ci-dessous sont définis de la manière suivante :
- Le terme **"extrait de levure"** réfère au contenu des cellules de levure, ledit contenu étant obtenu par tout procédé d'extraction adapté connu de l'homme du métier. L'extrait de levure selon l'invention est obtenu par un procédé sélectionné dans le groupe comprenant la plasmolyse, l'autolyse, la combinaison de la plasmolyse et de l'autolyse, de préférence l'autolyse. Avantageusement, une enzyme protéolytique peut être ajoutée lors du procédé d'extraction pour augmenter l'efficacité dudit procédé.
- Le terme **"fraction de levures"** recouvre des substances obtenues par séparation de l'enveloppe et du reste de la cellule de levure ; par exemple la fraction "écorces de levures" correspond aux enveloppes des cellules de levures à l'exclusion du contenu des cellules ; la fraction "extrait de levures" correspond au contenu des cellules de levures à l'exclusion des enveloppes.
- Le terme **"levures inactives"** réfère à des levures tuées, par tout procédé physique, chimique ou physico-chimique. Le plus souvent, les levures sont tuées par choc thermique au terme du processus de production, puis séchées.
- Le terme **"dérivés de levures"** recouvre l'ensemble des produits à base de levures : fractions, levures inactives, et d'autres compositions.
- Le terme **"mycorhize"** réfère à une association symbiotique entre des filaments mycéliens de champignons et des racines de plantes. Les filaments externes du mycélium se combinent aux racines des plantes et constituent ainsi un véritable prolongement du système racinaire qui va explorer le sol dans la périphérie de la racine. Le réseau mycélien dans le sol peut ainsi atteindre plusieurs millions de km/hectare, multipliant la zone de prospection des racines par 20 à 25 fois. Le mycélium n'est pas cloisonné, fluidifiant ainsi les transferts. Par extension, le terme mycorhize recouvre ici le terme champignon mycorhizien.
- Le terme **"propagule"** est utilisé pour désigner à la fois les spores, les vésicules et les fragments de racines contenant des vésicules, puisque toutes ces structures servent à propager l'espèce. En effet, le champignon mycorhizien forme des spores (isolées ou regroupées en sporocarpes) destinées à propager et disséminer l'espèce. Chez certaines espèces, des structures de reproduction, appelées vésicules intraracinaires, se différencient dans le cortex racinaire et possèdent des propriétés analogues à celles des spores (Les mycorhizes, La nouvelle révolution verte J. Fortin, C. Plenchette, Y. Piché 2008).
- Le terme **"substrat"** réfère à un support de culture à savoir un ensemble de produits destinés à servir de milieu de culture à certains végétaux. Leur mise en œuvre aboutit à la formation de milieux possédant une porosité en air et en eau telle qu'ils sont capables à la fois d'ancrer les organes absorbants des plantes et de leur permettre d'être en contact avec les solutions nécessaires à leur croissance. Ils sont généralement composés de matières organiques et de matières inorganiques. Ils sont généralement composé de tourbe (matière organique souvent majoritaire), d'autres matériaux organiques (notamment fibres de coco, écorces, fibres de bois, composts de déchets verts), de matériaux inorganiques (sables, pouzzolane, argiles, laines minérales, perlite, vermiculite). Dans la présente description, le terme **"substrat"** non suivi d'un adjectif se rapporte à un produit qui ne contient ni mycorhize, ni extrait de levure ; le terme **"substrat inoculé"** concerne un substrat dans lequel au moins une mycorhize a été ajoutée ; le terme **"substrat enrichi"** concerne un système comprenant un substrat, au moins une mycorhize et au moins un extrait de levure.
- Le terme **"taux de mycorhization"** représente le niveau d'infection mycorhizienne des racines de la plante observée. Il existe deux méthodes différentes d'évaluation de l'infection mycorhizienne : la méthode de Giovannetti et Mosse (1980) et celle de Trouvelot *et al.* (1986). La méthode de Giovannetti et Mosse est particulièrement adaptée à l'évaluation rapide de l'infection mycorhizienne de racines et propose un taux de mycorhization. La seconde méthode Trouvelot *et al,* plus complète mais plus longue, permet de calculer le taux de mycorhization, le taux de mycorhization ainsi que la richesse en arbuscules et vésicules d'un échantillon. C'est cette méthode qui a été utilisée pour les essais décrits par la Demanderesse.
- Le terme **"non-légumineuses"** signifie une plante dont le fruit n'est pas une gousse et qui n'appartient pas à la famille des Fabacées.
- Le terme **"biomasse"** signifie l'ensemble de la matière, organique et minérale, constituant une plante.
- Le terme **"environ"** placé devant une valeur chiffrée signifie plus ou moins 10% de cette valeur chiffrée.

### DESCRIPTION

Ainsi, la présente invention concerne une méthode d'amélioration de la croissance et/ou du développement et de la productivité de plantes non-légumineuses comprenant l'administration ou l'apport d'une composition comprenant au moins une mycorhize et au moins un extrait de levure.

Selon l'invention, l'amélioration de la croissance et/ou du développement et de la productivité de cultures de non-légumineuses inclut d'améliorer au moins un des paramètres suivants : le taux de mycorhization, l'enracinement, la croissance des plantes, la hauteur des plantes, la floraison, notamment en quantité ou en précocité, la biomasse fraiche, la biomasse sèche, le rendement, la nutrition minérale, la nutrition hydrique ou la résistance aux stress abiotiques, notamment au stress hydrique.

Selon un mode de réalisation de la présente invention, la méthode ne comprend pas l'addition de compost ou d'extrait de compost.

L'invention est applicable à tout type de plantes non-légumineuse, et notamment aux graminées et dicotylédones, aux plantes annuelles, bisannuelles et pérennes, aux légumes, aux céréales dont le blé, l'orge, le riz, le maïs, l'épeautre, l'avoine, le fornio, le seigle, le sorgho (notamment sorgho Friggo) et le millet, aux oléagineux, aux pommes de terre, à la canne à sucre, aux bananes, aux ananas, au cacao, au café, au tabac, aux plantes ligneuses, aux arbres fruitiers ou non, aux vignes, aux végétaux d'ornement (notamment géranium Zellino^{®} Rose Fluo).

Selon l'invention, les mycorhizes comprennent une souche active de champignons endomycorhiziens et/ou ectomycorhiziens. De préférence, les mycorhizes utilisées dans le procédé de l'invention comprennent une ou plusieurs souches actives d'un champignon endomycorhizien, plus préférentiellement du champignon endomycorhizien de l'ordre des *Glomerales.* Parmi les Glomérales, on peut citer le genre *Glomus sp* (nouvellement nommée *Sclérocystis sp ;* Schüßler et Walker, 2010), et plus précisément la souche *Glomus sp* codée LPA Val1, principe actif du produit Solrize^{®} développé et commercialisé par la société Agrauxine. Le produit Solrize^{®} se présente sous forme de granulé et contient le champignon endomycorhizien *Sclérocystis sp (ex Glomus sp)* à une concentration minimale de 10 propagules par gramme.

L'extrait de levure utilisé dans la présente invention est obtenu par plasmolyse ou autolyse, ou combinaison de la plasmolyse et de l'autolyse de levures, notamment du genre *Saccharomyces, Kluyveromyces, Candida* ou *Torula,* préférentiellement *S. cerevisiae.*

Selon un autre mode de réalisation, les méthodes d'extraction des levures peuvent être renforcées par l'ajout d'additifs comme les enzymes, notamment les enzymes protéolytiques ou des composés chimiques, notamment les sulfites.

Les extraits de levures utilisés dans l'invention peuvent provenir de toutes espèces de levures, en particulier de levures du genre Saccharomyces, notamment *S. cerevisiae.* Plus particulièrement, les extraits de levure sont du type de ceux commercialisés par la société Agro-Levures et Dérivés.

L'invention comprend également une composition comprenant au moins une mycorhize et au moins un extrait de levure, dans laquelle le rapport massique mycorhize / extrait de levure est compris entre 0,01 et 100.

On peut ainsi envisager une composition consistant en un substrat complémenté avec au moins une mycorhize et au moins un extrait de levure, dans lequel on peut planter directement les plantes non-légumineuses.

Selon un mode de réalisation de la méthode de l'invention, le rapport massique mycorhize/extrait de levure est compris entre 0,01 et 100. Selon un mode de réalisation de l'invention, le rapport massique mycorhize/extrait de levure est compris préférentiellement entre 0,05 et 20, plus préférentiellement entre 0,1 et 10. Dans un autre mode de réalisation, le rapport massique mycorhize/extrait de levure est égal à environ 16, environ 8, environ 4 ou environ 2.

La quantité de mycorhizes dans la composition de l'invention est comprise entre 0,1 et 15 kg/m³, préférentiellement entre 0,5 et 8 kg/m³, plus préférentiellement entre 1 et 4 kg/m³ de composition.

Dans un autre mode de réalisation, la quantité de mycorhizes dans la composition est égale à environ 2 kg/m³, environ 4 kg/m³ ou environ 8 kg/m³.

La quantité d'extrait de levure dans la composition de l'invention est comprise entre 0,1 et 10 kg/m³, préférentiellement entre 0,2 et 5 kg/m³, plus préférentiellement entre 0,4 et 2 kg de matière sèche par m³ de substrat. Selon un autre mode de réalisation, la quantité d'extrait de levure est d'environ 0,5 kg/m³ ou d'environ 1 kg/m³ de substrat enrichi.

Selon un mode de réalisation particulier, la composition de la présente invention ne comprend pas de compost ou d'extrait de compost. Suivant un mode de réalisation, la composition de l'invention ne comprend pas de bactérie.

Suivant un mode de réalisation, la composition selon l'invention pourra être sous forme de poudre mouillable (WP), de granulé (WG) ou de liquide.

Selon un autre mode de réalisation, l'extrait de levure et la mycorhize sont administrés simultanément ou successivement, par application au sol (pulvérisation, épandage, arrosage, ferti-irrigation, goutte à goutte, dans la raie de semis ou en plein), par trempage de racine, par traitement de semences ou par incorporation à un support de cultures ou par tout moyen permettant la mise en contact immédiate ou future de la composition avec les racines à inoculer.

Selon un mode de réalisation, la mycorhize et l'extrait de levure utilisés dans la présente invention sont mélangés dans un même container ou placés dans deux containers séparés.

Selon un mode de réalisation de l'invention, la quantité de mycorhizes apportée par hectare est comprise entre 0,1 et 100 kg/ha, préférentiellement entre 0,3 et 50 kg/ha, plus préférentiellement entre 0,5 et 20 kg/ha.

Dans un autre mode de réalisation, la quantité d'extrait de levure apportée par hectare selon l'invention est comprise entre 0,1 et 50 kg/ha, préférentiellement entre 0,5 et 20 kg/ha, plus préférentiellement entre 1 et 10 kg de matière sèche par hectare.

Selon un mode de réalisation, la composition selon l'invention comprend au moins une mycorhize et au moins un extrait de levure et un substrat.

Dans ce mode de réalisation, la composition se présente préférentiellement sous forme de solide, notamment de solide particulaire, et dans certains modes de réalisation, de poudre.

Avantageusement, ledit substrat comprend de façon non limitative de la tourbe et/ou de l'argile pure, du sable, de la pouzzolane, de la perlite, de la fibre de bois, de la fibre de coco, de la tourbe blonde, de la tourbe noire, de la terre de bruyère, des écorces, de la vermiculite, de la magnésie, de la chaux, de la laine. Ledit substrat peut être un amendement organique, un substrat plantation, un substrat fleurissement, ou tout autre substrat d'agriculture. Suivant un mode de réalisation, le substrat est un support de culture du type de ceux commercialisés par les spécialistes pour les horticulteurs professionnels.

Un objet de l'invention est donc un milieu de plantation ou de culture d'une plante non légumineuse comprenant au moins une mycorhize et au moins un extrait de levure et optionnellement du substrat.

L'invention concerne également un procédé de fabrication d'une composition selon l'invention comprenant, au moins une mycorhize et au moins un extrait de levure, dans lequel on mélange ladite mycorhize et ledit extrait de levure. L'invention concerne également un procédé de fabrication d'un substrat enrichi selon l'invention, qui comprend l'incorporation simultanément ou successivement de mycorhizes et d'extraits de levure dans le substrat.

L'invention concerne également un procédé de fabrication d'un substrat enrichi selon l'invention, comprenant dans une première étape le mélange de mycorhizes avec un substrat, suivie de l'ajout d'au moins un extrait de levure.

Selon un mode de réalisation de la présente invention, l'amélioration du taux de mycorhization des plantes placées dans le substrat enrichi signifie que la mycorhization est augmentée de 10 à 150%, de préférence de 12 à 90%, plus préférentiellement de 15 à 70% par rapport à la mycorhization obtenue si la plante est placée dans un substrat non enrichi.

Selon un autre mode de réalisation, l'amélioration de la hauteur des plantes placées dans le substrat enrichi signifie que la hauteur des plantes est augmentée de 10 à 150%, de préférence de 12 à 110%, plus préférentiellement de 15 à 80% par rapport à la hauteur de la plante observée si celle-ci est placée dans un substrat non enrichi.

Selon un autre mode de réalisation, l'amélioration de la biomasse fraîche et sèche des plantes placées dans le substrat enrichi signifie que la biomasse fraîche ou sèche est augmentée de 5 à 250%, de préférence de 20 à 200%, plus préférentiellement de 30 à 160% par rapport à la biomasse fraîche ou sèche obtenue si la plante est placée dans un substrat non enrichi.

Selon un autre mode de réalisation, l'amélioration de la floribondité des plantes placées dans le substrat enrichi signifie que le nombre de boutons floraux est augmenté de 10 à 150%, de préférence de 15 à 100%, plus préférentiellement de 20 à 80% par rapport aux nombres de boutons floraux observée si la plante est placée dans un substrat non enrichi.

### EXEMPLES

La présente invention se comprendra mieux à la lecture des exemples suivants qui illustrent non-limitativement l'invention.

### 1. Matériels et méthodes

Les matériels et méthodes sont communs aux quatre exemples présentés.

***Nombre d'essais** :* Quatre essais ont été réalisés, un sur Géranium (Exemple 1) et deux sur Sorgho (Exemples 2 et 3) et un sur Chrysanthème (Exemple 4).

***Lieu** :* Les essais ont été réalisés sous serre, à Angers, France.

***Matériel végétal** :* Les tests ont été réalisés sur trois types de végétaux : le Sorgho Friggo, le Géranium Zellino^{®} Rose Fluo et le Chrysanthème.

### Produits de Levures :

- Extraits de levure : Extrait Lev1 et Extrait Lev2
- Levures inactives : INACT1

***Mycorhize** :* Les mycorhizes utilisées correspondent au produit SOLRIZE^{®} PRO commercialisé par la société Agrauxine. Elles contiennent une souche active du champignon endomycorhizien *Glomus sp.*

***Substrat** :* Le substrat utilisé a la composition suivante : argile pure, sable, pouzzolane, perlite, tourbe.

### Dispositif et modalités de l'essai :

- 4 répétitions par modalité avec une plante par pot/répétition soit 4 plantes par modalité.
- Les mycorhizes utilisées (Solrize^{®} Pro) ont été testées à 1, 2 ou 3 doses : DN (8 kg/m³), DN/2 (4 kg/m³), DN/3 (2,7 kg/m³).
- Les extraits de levure (Extrait Lev1 et Extrait Lev2) ont été testés à 1kg/m³.
- La levure inactive (INACT1) a été testée à 1 kg/m³.

### Évaluation des essais :

Les essais seront évalués par la mesure de paramètres quantitatifs suivants :
- Taux de mycorhization du système racinaire ;
- Mesure de la hauteur des plantes (sauf exemple 1) ;
- Poids de la biomasse totale, fraîche et sèche (sauf exemple 1) ;
- Nombre de boutons floraux (uniquement exemple 4).

### 2. Résultats

Les mycorhizes sont mélangés manuellement au substrat à des concentrations de 8, 4 ou 2,7 kg/m³ de substrat. Les extraits de levure (Extrait Lev1 et Extrait Lev2) ou les levures inactives (INACT1) sont ensuite ajoutés au substrat inoculé.

### EXEMPLE 1 (Géranium) : Comparaison extrait de levures vs levures inactives

### Effet sur le taux de mycorhization (TM) en %.

**Tableau 1A : Évaluation du taux de mycorhization du géranium.**

| Dose de Solrize : | DN | DN/2 |
|---|---|---|
| Solrize^{®} Pro seul | 26,9 | 21,0 |
| Solrize^{®} Pro + Extrait Lev1 (1 kg/m³) | 37,0 | 27,6 |
| Solrize^{®} Pro + INACT1 (1 kg/m³) | 18,5 | 12,8 |
| *Extrait Lev1 1kg : Augmentation TM* / *Solrize* | 37% | 31% |
| *INACT1 1kg : Augmentation TM*/*Solrize* | -31% | -39% |

Le tableau 1A montre que l'association d'extraits de levure (Extrait Lev1) et de mycorhizes augmente significativement le taux de mycorhization du géranium (+ 31-37%). A l'opposé, les levures inactives montrent un effet négatif sur le taux de mycorhization du géranium. Aucune mycorhization n'est observée lorsque l'on utilise un extrait de levure seul.

### EXEMPLE 2 (Sorgho) : Comparaison entre extrait de levures et levures inactives

### Effet sur le taux de mycorhization (TM) en %.

**Tableau 2A : Évaluation du taux de mycorhization du Sorgho.**

| Dose de Solrize : | DN | DN/2 |
|---|---|---|
| Solrize^{®} Pro seul | 28,8 | 21,2 |
| Extrait Lev 1 seul (1 kg/m³)* | 0 | 0 |
| Solrize^{®} Pro + Extrait Lev1 (1 kg/m³) | 48,4 | 34,4 |
| Solrize^{®} Pro + Extrait Lev1 (0,5 kg/m³) | 38,5 | 29,8 |
| Solrize^{®} Pro + INACT1 (1 kg/m³) | 10,6 | 14,2 |
| *Extrait Lev1 1kg : Augmentation TM* / *Témoin* | 68% | 62% |
| *INACT1 1kg : Augmentation TM* / *Témoin* | -63% | -33% |
| *cette ligne correspond aux résultats observés pour l'extrait de levure seul sans Solrize^{®} Pro. | | |

Le tableau 2A confirme les résultats observés sur géranium : l'association d'extraits de levure (Extrait Lev1) et de mycorhizes augmente significativement le taux de mycorhization du sorgho (+ 62-68%). A l'opposé, les levures inactives montrent un effet négatif sur le taux de mycorhization du sorgho.

L'extrait de levure seul (ou l'absence de produit, témoin négatif) ne permet pas d'observer de mycorhization. On observe également une augmentation de la mycorhization lorsque l'on utilise des doses plus faibles d'extrait de levure.

### Effet sur la hauteur des plantes (HP) en cm, à 6 semaines, et à 10 semaines.

**Tableau 2B : Évaluation de la hauteur des plants de sorgho, 6 semaines et 10 semaines après semis.**

| Dose de Solrize | 6 semaines après semis | | 10 semaines après semis | |
|---|---|---|---|---|
| | DN | DN/2 | DN | DN/2 |
| Solrize^{®} Pro | 29,8 | 33,2 | 48,9 | 46,8 |
| Extrait Lev 1 seul (1 kg/m³)* | | | 53.,5 | 53,5 |
| Solrize^{®} Pro + Extrait Lev1 (1 kg/m³) | 41,3 | 41,0 | 64,8 | 58,2 |
| Solrize^{®} Pro + INACT1 (1 kg/m³) | 36,0 | 38,7 | 50,9 | 49,9 |
| *Extrait Lev1 1kg : Augmentation TM*/ *Solrize* | 38% | 24% | 33% | 24% |
| *INACT1 1kg : Augmentation TM* / *Solrize* | 21% | 17% | 4% | 7% |
| *cette ligne correspond aux résultats observés pour l'extrait de levure seul sans Solrize^{®} Pro. | | | | |
| Le tableau 2B montre que l'association d'extraits de levure (Extrait Lev1) et de mycorhizes augmente significativement la croissance (hauteur) du sorgho (+ 24 - 38%). Cet effet surprenant observé est beaucoup plus important que celui observé avec l'association de levures inactives et de mycorhizes. | | | | |

Par ailleurs, on observe un effet de synergie lorsque l'on utilise l'extrait de levure en combinaison avec le mycorhize.

### Effet sur la hauteur des plantes (HP) en cm, à 10 semaines.

| Dose de Solrize | 10 semaines après semis | |
|---|---|---|
| | DN | DN/2 |
| Témoin négatif | 49,3 | 49,3 |
| Solrize^{®} Pro | 48,9 | 46,8 |
| Extrait Lev 1 seul (0,5 kg/m³)* | 48,0 | 48,0 |
| Solrize^{®} Pro + Extrait Lev1 (0,5 kg/m³) | 55,1 | 46,5 |

On observe aussi un effet de synergie lorsque l'on utilise l'extrait de levure à une dose inférieure, en combinaison avec le mycorhize à la dose habituelle (DN).

### Poids de biomasse fraiche (BF) et sèche (BS), en g.

**Tableau 2C : Évaluation de biomasse fraiche et sèche du sorgho, 10 semaines après semis.**

| Dose de Solrize | **BF** | | **BS** | |
|---|---|---|---|---|
| | DN | DN/2 | DN | DN/2 |
| Solrize^{®} Pro seul | 3,4 | 3,9 | 0,7 | 0,8 |
| Extrait Lev 1 seul (1 kg/m³)* | 6,0 | 6,0 | 1,1 | 1,1 |
| Solrize^{®} Pro + Extrait Lev1 (1 kg/m³) | 8,0 | 8,4 | 1,6 | 1,8 |
| Solrize^{®} Pro + INACT1 (1 kg/m³) | 5,7 | 6,5 | 1,2 | 1,2 |
| *Extrait Lev1 1kg : Augmentation TM*/ *Solrize* | 139% | 118% | 129% | 124% |
| *INACT1 1kg : Augmentation TM* / *Solrize* | 62% | 67% | 71% | 50% |
| *cette ligne correspond aux résultats observés pour l'extrait de levure seul sans Solrize^{®} Pro. | | | | |
| Le tableau 2C montre que l'association d'extraits de levure (Extrait Lev1) et de mycorhizes augmente très significativement la biomasse fraiche et sèche du sorgho (+ 118-145%). Cet effet surprenant observé est beaucoup plus important que celui observé avec l'association de levures inactives et de mycorhizes (+ 59-71%). | | | | |

### EXEMPLE 3 (Sorgho) : Comparaison de deux extraits de levures

### Effet sur le taux de mycorhization (TM) en %.

**Tableau 3A : Évaluation du taux de mycorhization du Sorgho.**

| Dose de Solrize : | DN |
|---|---|
| Solrize^{®} Pro | 27,9 |
| Solrize^{®} Pro + Extrait Lev1 (1 kg/m³) | 38,5 |
| Solrize^{®} Pro + Extrait Lev2 (1 kg/m³) | 35,6 |
| *Extrait Lev1 1kg : Augmentation TM* / *Témoin* | *38%* |
| *Extrait Lev2 1kg : Augmentation TM* / *Témoin* | *28%* |

Le tableau 3A confirme les résultats observés dans les exemples 1 et 2 : l'association d'extraits de levure (Extrait Lev1 ou Extrait Lev2) et de mycorhizes augmente significativement le taux de mycorhization du sorgho (+ 28-38 %).

### Effet sur la hauteur des plantes (HP) en cm, à 10 semaines.

**Tableau 3B : Évaluation de la hauteur des plants de sorgho, 10 semaines après semis.**

| Dose de Solrize | 6 semaines après semis |
|---|---|
| | DN |
| Solrize^{®} Pro | 54,4 |
| Solrize^{®} Pro + Extrait Lev1 (1 kg/m³) | 68,4 |
| Solrize^{®} Pro + Extrait Lev2 (1 kg/m³) | 66,1 |
| *Extrait Lev1 1kg : Augmentation HP* / *Témoin* | *26%* |
| *Extrait Lev2 1kg : Augmentation HP* / *Témoin* | *22%* |

Le tableau 3B confirme les résultats observés dans l'exemple 2 : l'association des extraits de levure (Extrait Lev1 ou Extrait Lev2) et de mycorhizes augmente significativement la croissance (hauteur) du sorgho (+ 22-26%).

### Poids de biomasse fraiche (BF) et sèche (BS), en g.

**Tableau 3C : Évaluation de biomasse fraiche et sèche du sorgho, 10 semaines après semis.**

| Dose de Solrize | **Biomasse fraiche (BF) en g** | **Biomasse sèche (BS) en g** |
|---|---|---|
| | DN | DN |
| Solrize^{®} Pro | 7,7 | 1,9 |
| Solrize^{®} Pro + Extrait Lev1 (1 kg/m³) | 13,1 | 3,1 |
| Solrize^{®} Pro + Extrait Lev2 (1 kg/m³) | 12,4 | 2,9 |
| *Extrait Lev1 1kg : Augmentation BF & BS* / *Témoin* | *70%* | *63%* |
| *Extrait Lev2 1kg : Augmentation BF & BS* / *Témoin* | *61%* | *53%* |

Le tableau 3C confirme les résultats observés dans l'exemple 2 : l'association des extraits de levure (Extrait Lev1 ou Extrait Lev2) et de mycorhizes augmente significativement la biomasse fraiche et sèche du sorgho (+53-70%).

En conclusion, les deux extraits de levures testés ont tous les deux montré des effets très intéressants en association avec des mycorhizes.

### EXEMPLE 4 (Chrysanthème) :

### Effet sur le taux de mycorhization (TM) en %.

**Tableau 4A : Evaluation du taux de mycorhization du Chrysanthème.**

| Dose de Solrize | DN | DN/2 | DN/3 |
|---|---|---|---|
| Solrize^{®} Pro | 29,0 | 22,1 | 18,8 |
| Solrize^{®} Pro + Extrait Lev1 (1 kg/m³) | 39,5 | 31,9 | 28,0 |
| *Extrait Lev1 1kg : Augmentation TM* / *Témoin* | 36% | 44% | 49% |

Le tableau 4A confirme les résultats observés sur géranium et sorgho : l'association d'extraits de levure (Extrait Lev1) et de mycorhizes augmente significativement le taux de mycorhization du chrysanthème au bout de 14 semaines (+ 36-49%), et permet de réduire la dose de mycorhize jusqu'à DN/3 tout en conservant un bon taux de mycorhization. L'utilisation de l'extrait de levure seul ne permet pas d'observer de mycorhization.

### Effet sur la hauteur des plantes (HP) en cm, à 10 semaines.

**Tableau 4B : Évaluation de la hauteur des plants de chrysanthème, à 10 semaines.**

| Dose de Solrize | DN | DN/2 | DN/3 |
|---|---|---|---|
| Solrize^{®} Pro | 12,3 | 10,5 | 11,3 |
| Solrize^{®} Pro + Extrait Lev1 (1 kg/m³) | 17,5 | 14,8 | 17,3 |
| *Extrait Lev1 1kg : Augmentation TM* / *Témoin* | *42%* | *41%* | *53%* |

Le tableau 4B confirme les résultats observés sur géranium et sorgho : l'association d'extraits de levure (Extrait Lev1) et de mycorhizes augmente significativement la croissance (hauteur) du chrysanthème (+ 41-53%) au bout de 14 semaines.

### Poids de biomasse fraiche (BF) et sèche (BS), en g.

**Tableau 4C : Évaluation de biomasse fraiche et sèche du chrysanthème, à 10 semaines.**

| Dose de Solrize | **Biomasse fraiche (BF) en g** | | | **Biomasse sèche (BS) en g** | | |
|---|---|---|---|---|---|---|
| | DN | DN/2 | DN/3 | DN | DN/2 | DN/3 |
| Solrize^{®} Pro | 29,6 | 29,0 | 26,3 | 5,7 | 5,5 | 4,9 |
| Extrait Lev1 (1 kg/m³) | 29,6 | 29,6 | 29,6 | 7,1 | 7,1 | 7,1 |
| Solrize^{®} Pro + Extrait Lev1 (1 kg/m³) | 31,3 | 31,8 | 30,2 | 7,6 | 7,2 | 6,8 |
| *Extrait Lev1 1kg : Augmentation BF & BS* / *Témoin* | *6%* | *10%* | *15%* | *33%* | *31%* | *39%* |

Le tableau 4C confirme les résultats observés sur géranium et sorgho : l'association d'extraits de levure (Extrait Lev1) et de mycorhizes augmente significativement la biomasse fraiche et sèche du chrysanthème (+ 6-39%).

### Effet sur la floraison (nombre de boutons floraux).

**Tableau 4D : Évaluation du nombre de boutons floraux du chrysanthème.**

| Dose de Solrize | DN | DN/2 | DN/3 |
|---|---|---|---|
| Solrize^{®} Pro | 2 | 2,5 | 1,5 |
| Solrize^{®} Pro + Extrait Lev1 (1 kg/m3) | 3 | 3,8 | 3,5 |
| *Extrait Lev1 1kg : Augmentation TM* / *Témoin* | *50%* | *52%* | *133%* |

Le tableau 4D montre que l'association d'extraits de levure (Extrait Lev1) et de mycorhizes augmente significativement le nombre de boutons floraux du chrysanthème (plus de 50 %).

### Exemple 5 : Hibiscus

### Effet sur la floraison (nombre de boutons floraux)

**Tableau 5 : Évaluation du nombre de boutons floraux de l'hibiscus**

| Dose de Solrize (en kg/m³) | 1,2 | 1,0 |
|---|---|---|
| Solrize^{®} Pro | 3,7 | 3,3 |
| Extrait Lev1 (1 kg/m3) | 5,3 | 5,3 |
| Solrize^{®} Pro + Extrait Lev1 (1 kg/m3) | 8 | 6,7 |

Le tableau 5 montre que l'association d'extraits de levure (Extrait Lev1) et de mycorhizes augmente significativement le nombre de boutons floraux de l'hibiscus.

## Revendications

1. Méthode d'amélioration de la croissance et/ou du développement et de la productivité de plantes non-légumineuses comprenant l'apport d'une composition comprenant au moins une mycorhize et au moins un extrait de levure, correspondant au contenu des cellules de levures à l'exclusion des enveloppes et obtenu par un procédé sélectionné dans le groupe comprenant la plasmolyse, l'autolyse, et la combinaison de la plasmolyse et de l'autolyse.

2. Méthode selon la revendication 1, dans laquelle ladite amélioration de la croissance et/ou du développement de cultures de non légumineuses inclut d'améliorer au moins un des paramètres suivant : le taux de la mycorhization, l'enracinement, la croissance des plantes, la hauteur des plantes, la floraison, la biomasse fraiche, la biomasse sèche, le rendement, la nutrition minérale, la nutrition hydrique ou la résistance aux stress abiotiques.

3. Méthode selon la revendication 2 dans laquelle ledit stress abiotique est le stress hydrique.

4. Méthode selon l'une quelconque des revendications 1 à 3, dans laquelle lesdites non-légumineuses sont choisies dans le groupe constitué des graminées, des dicotylédones, des plantes annuelles, bisannuelles et pérennes, des légumes, des céréales, des oléagineux, des pommes de terre, des cannes à sucre, des bananes, des ananas, du cacao, du café, du tabac, des plantes ligneuses, des arbres fruitiers ou non, des vignes, et des végétaux d'ornement.

5. Méthode selon la revendication 4, dans laquelle lesdites graminées sont choisies parmi le blé, l'orge, le riz, le maïs, l'épeautre, l'avoine, le fornio, le seigle, le sorgho et le millet.

6. Méthode selon la revendication 4, dans laquelle lesdits végétaux d'ornement sont des géraniums.

7. Méthode selon l'une quelconque des revendications 1 à 6, dans laquelle ladite mycorhize comprend une souche active de champignon endomycorhizien.

8. Méthode selon la revendication 7, dans laquelle ladite souche active de champignon endomycorhizien est un champignon endomycorhizien *Sclérocystis sp.*

9. Méthode selon l'une quelconque des revendications 1 à 8, dans laquelle ledit extrait de levure est obtenu par plasmolyse ou autolyse, ou combinaison de la plasmolyse et de l'autolyse de levures, notamment du genre Saccharomyces, Kluyveromyces, Candida ou Torula, préférentiellement S. *cerevisiae.*

10. Méthode selon la revendication 9, dans laquelle lesdites levures sont du genre Saccharomyces, Kluyveromyces, Candida ou Torula.

11. Méthode selon la revendication 10, dans laquelle lesdites levures sont des S. *cerevisiae.*

12. Méthode selon l'une quelconque des revendications 1 à 11, dans laquelle l'extrait de levure et la mycorhize sont administrés simultanément ou successivement, par application au sol.

13. Méthode selon la revendication 12, dans laquelle l'application au sol est choisie dans le groupe constituée des pulvérisation, épandage, arrosage, ferti-irrigation, goutte à goutte, dans la raie de semis ou en plein, trempage de racine, traitement de semences ou par incorporation à un support de cultures et tout moyen permettant la mise en contact immédiate ou future de la composition avec les racines à inoculer.

14. Composition comprenant au moins une mycorhize et au moins un extrait de levure, correspondant au contenu des cellules de levures à l'exclusion des enveloppes et obtenu par un procédé sélectionné dans le groupe comprenant la plasmolyse, l'autolyse, et la combinaison de la plasmolyse et de l'autolyse, dans laquelle le rapport massique mycorhize / extrait de levure est compris entre 0,01 et 100.

15. Composition selon la revendication 14, dans laquelle ladite mycorhize et ledit extrait de levure sont mélangés dans un même container ou placés dans deux containers séparés.

16. Composition selon la revendication 14 ou la revendication 15, comprenant en outre un substrat.

17. Composition selon l'une quelconque des revendications 14 à 16, dans laquelle la quantité de mycorhizes est comprise entre 0,1 et 15 kg/m³ de la composition.

18. Composition selon la revendication 17, dans laquelle la quantité de mycorhize est comprise entre 0,5 et 8 kg/m³ de la composition.

19. Composition selon la revendication 17, dans laquelle la quantité de mycorhize est comprise entre 1 et 4 kg/m³ de la composition.

20. Composition selon l'une quelconque des revendications 14 à 19, dans laquelle la quantité d'extrait de levure est comprise entre 0,1 et 10 kg/m³ de la composition.

21. Composition selon la revendication 20, dans laquelle la quantité d'extrait de levure est comprise entre 0,2 et 5 kg/m³ de la composition.

22. Composition selon la revendication 20, dans laquelle la quantité d'extrait de levure est comprise entre 0,4 et 2 kg/m³ de la composition.

23. Composition selon l'une quelconque des revendications 14 à 22, dans laquelle le substrat comprend des matières organiques, et des matières inorganiques.

24. Composition selon la revendication 23, dans laquelle les matières organiques sont de la tourbe, ou les matières inorganiques sont choisies parmi le sable, l'argile.

25. Utilisation de la composition selon l'une des revendications 14 à 24 pour améliorer la croissance et/ou le développement et de la productivité de plantes non-légumineuses.

26. Utilisation selon la revendication 25 **caractérisée en ce que** l'apport en mycorhize est compris entre 0,1 et 100 kg/ha.

27. Utilisation selon la revendication 25 **caractérisée en ce que** l'apport en mycorhize est compris entre 0,3 et 50 kg/ha.

28. Utilisation selon la revendication 25 **caractérisée en ce que** l'apport en mycorhize est compris entre 0,5 et 20 kg/ha.

29. Utilisation selon l'une quelconque des revendications 25 à 28 **caractérisée en ce que** l'apport en extrait de levure est compris entre 0,1 et 50 kg/ha.

30. Utilisation selon l'une quelconque des revendications 25 à 28, **caractérisée en ce que** l'apport en extrait de levure est compris entre 0,5 et 20 kg/ha.

31. Utilisation selon l'une quelconque des revendications 25 à 28, **caractérisée en ce que** l'apport en extrait de levure est compris entre 1 et 10 kg/ha.

32. Procédé de fabrication d'une composition selon l'une quelconque des revendications 14 à 24 comprenant l'incorporation simultanément ou successivement d'au moins une mycorhize et d'au moins un extrait de levure, correspondant au contenu des cellules de levures à l'exclusion des enveloppes et obtenu par un procédé sélectionné dans le groupe comprenant la plasmolyse, l'autolyse, et la combinaison de la plasmolyse et de l'autolyse, avec un substrat.

## Patentansprüche

1. Verfahren zur Verbesserung des Wachstums und/oder der Entwicklung und der Produktivität von Nicht-Leguminosen, umfassend die Bereitstellung einer Zusammensetzung, die mindestens eine Mykorrhiza und mindestens einen Hefeextrakt umfasst, der dem Inhalt der Hefezellen unter Ausschluss der Schalen entspricht und durch ein Verfahren erhalten wird, das aus der Gruppe ausgewählt ist, die Plasmolyse, Autolyse und die Kombination von Plasmolyse und Autolyse umfasst.

2. Verfahren nach Anspruch 1, bei dem die Verbesserung des Wachstums und/oder der Entwicklung von nicht-leguminösen Pflanzenkulturen die Verbesserung mindestens eines der folgenden Parameter umfasst: den Mykorrhizierungsgrad, die Bewurzelung, das Wachstum der Pflanzen, die Höhe der Pflanzen, die Blüte, die frische Biomasse, die trockene Biomasse, den Ertrag, die mineralische Ernährung, die hydrische Ernährung oder die Resistenz gegen abiotischen Stress.

3. Verfahren nach Anspruch 2, bei dem der abiotische Stress ein hydrischer Stress ist.

4. Verfahren nach einem der Ansprüche 1 bis 3, bei dem die nicht-leguminösen Pflanzen aus der Gruppe ausgewählt werden, die aus Gräsern, Dikotyledonen, ein-, zwei- und mehrjährigen Pflanzen, Gemüse, Getreide, ölproduzierenden Pflanzen, Kartoffeln, Zuckerrohr, Bananen, Ananas, Kakao, Kaffee, Tabak, Holzpflanzen, Obst- oder Nichtfruchtbäumen, Weinreben, Zierpflanzen besteht.

5. Verfahren nach Anspruch 4, bei dem das genannte Getreide aus der Gruppe Weizen, Gerste, Reis, Mais, Dinkel, Hafer, Fonio, Roggen, Sorghum und Hirse ausgewählt wird.

6. Verfahren nach Anspruch 4, bei dem die genannten Zierpflanzen Geranien sind.

7. Verfahren nach einem der Ansprüche 1 bis 6, bei dem die Mykorrhiza einen aktiven Stamm eines endomykorrhizalen Pilzes umfaßt.

8. Verfahren nach den Ansprüchen 7, bei dem der aktive Stamm des Endomykorrhizapilzes ein Endomykorrhizapilz Sclerocystis sp. ist.

9. Verfahren nach einem der Ansprüche 1 bis 8, bei dem der Hefeextrakt durch Plasmolyse oder Autolyse oder eine Kombination aus Plasmolyse und Autolyse von Hefen, insbesondere der Gattung *Saccharomyces, Kluyveromyces, Candida oder Torula,* vorzugsweise *S*. *cerevisiae,* erhalten wird.

10. Verfahren nach Anspruch 9, bei dem die Hefen der Gattung *Saccharomyces, Kluyveromyces, Candida* oder *Torula* sind.

11. Verfahren nach Anspruch 10, bei dem die Hefe *S*. *cerevisiae* ist.

12. Verfahren nach einem der Ansprüche 1 bis 11, bei dem der Hefeextrakt und die Mykorrhiza gleichzeitig oder nacheinander durch Aufbringen auf den Boden verabreicht werden.

13. Verfahren nach Anspruch 12, bei dem das Aufbringen auf den Boden aus der Gruppe ausgewählt wird, die besteht aus: Besprühen, Ausbringen , Bestreuen, Fertigation, tropfenweise, in der Sämaschine oder im Freiland, durch Eintauchen der Wurzeln, durch Behandlung des Saatguts oder durch Einarbeitung in einen Kulturträger oder durch jedes Mittel, das es ermöglicht, die Zusammensetzung sofort oder in Zukunft mit den zu beimpfenden Wurzeln in Kontakt zu bringen.

14. Zusammensetzung, die mindestens eine Mykorrhiza und mindestens einen Hefeextrakt umfasst, der dem Inhalt der Hefezellen unter Ausschluss der Schalen entspricht und durch ein Verfahren erhalten wird, das aus der Gruppe ausgewählt ist, die die Plasmolyse, die Autolyse und die Kombination aus Plasmolyse und Autolyse umfasst, wobei das Gewichtsverhältnis von Mykorrhiza zu Hefeextrakt zwischen 0,01 und 100 liegt.

15. Zusammensetzung nach Anspruch 14, in der die genannte Mykorrhiza und der genannte Hefeextrakt in demselben Behälter gemischt oder in zwei getrennten Behältern angeordnet sind.

16. Zusammensetzung nach Anspruch 14 oder Anspruch 15, die auch ein Substrat umfasst.

17. Zusammensetzung nach einem der Ansprüche 14 bis 16, in der die Menge an Mykorrhiza zwischen 0, 1 und 15 kg/m³ der Zusammensetzung liegt.

18. Zusammensetzung nach Anspruch 17, in der die Menge an Mykorrhizae zwischen 0,5 und 8 kg/m³ der Zusammensetzung liegt.

19. Zusammensetzung nach Anspruch 17, in der die Menge an Mykorrhizae zwischen 1 und 4 kg/m³ der Zusammensetzung liegt.

20. Zusammensetzung nach einem der Ansprüche 14 bis 19, in der die Menge an Hefeextrakt zwischen 0, 1 und 10 kg/m³ der Zusammensetzung liegt.

21. Zusammensetzung nach einem der Ansprüche 20, in der die Menge an Hefeextrakt zwischen 0,2 und 5 kg/m³ der Zusammensetzung beträgt.

22. Zusammensetzung nach Anspruch 20, in der die Menge an Hefeextrakt zwischen 0,4 und 2 kg/m³ der Zusammensetzung liegt

23. Zusammensetzung nach einem der Ansprüche 14 bis 22, bei der das Substrat organische Stoffe und anorganische Stoffe enthält.

24. Zusammensetzung nach einem der Ansprüche 22, in der die organische Substanz Torf ist oder die anorganische Substanz aus Sand, Ton ausgewählt ist.

25. Verwendung der Zusammensetzung nach einem der Ansprüche 14 bis 24 zur Verbesserung des Wachstums und/oder der Entwicklung und der Produktivität von nicht-Leguminosen Pflanzen.

26. Verwendung nach einem der Ansprüche 25, **dadurch gekennzeichnet, daß** die Mykorrhizaversorgung zwischen 0, 1 und 100 kg/ha liegt.

27. Verwendung nach Anspruch 25, **dadurch gekennzeichnet, daß** die Mykorrhizaversorgung zwischen 0,3 und 50 kg/ha liegt.

28. Verwendung nach Anspruch 25, **dadurch gekennzeichnet, daß** die Mykorrhizaversorgung zwischen 0,5 und 20 kg/ha liegt.

29. Verwendung nach einem der Ansprüche 25 bis 28, **dadurch gekennzeichnet, daß** die Hefeextraktversorgung zwischen 0, 1 und 50 kg/ha liegt.

30. Verwendung nach einem der Ansprüche 25 bis 28, **dadurch gekennzeichnet, daß** die Hefeextraktversorgung zwischen 0,5 und 20 kg/ha liegt.

31. Verwendung nach einem der Ansprüche 25 bis 28, **dadurch gekennzeichnet, daß** die Hefeextraktversorgung zwischen 1 und 10 kg/ha liegt.

32. Verfahren zur Herstellung einer Zusammensetzung nach einem der Ansprüche 14 bis 24, umfassend den gleich-zeitigen oder aufeinanderfolgenden Einbau mindestens einer Mykorrhiza und mindestens eines Hefeextrakts, der dem Inhalt an Hefezellen unter Ausschluß der Schalen entspricht und durch ein Verfahren erhalten wird, das aus der Gruppe ausgewählt ist, die die Plasmolyse, die Autolyse und die Kombination von Plasmolyse und Autolyse mit einem Substrat umfaßt.

## Claims

1. Method for improving the growth and/or development and the productivity of non-leguminous plants, comprising the provision of a composition comprising at least one mycorrhiza and at least one yeast extract corresponding to the content of the yeast cells with the exclusion of the shells and obtained by a process selected from the group comprising plasmolysis, autolysis, and the combination of plasmolysis and autolysis.

2. Method according to Claim 1, in which said improving of the growth and/or development of non-leguminous plant crops includes improving at least one of the following parameters: the degree of mycorrhization, the rooting, the growth of the plants, the height of the plants, the flowering, the fresh biomass, the dry biomass, the yield, the mineral nutrition, the hydric nutrition or the resistance to abiotic stresses.

3. Method according to Claim 2, in which said abiotic stress is hydric stress.

4. Method according to any one of Claims 1 to 3, in which said non-leguminous plants are selected in the group consisting of grasses, dicotyledons, annual, biennial and perennial plants, vegetables, cereals, oil-producing plants, potatoes, sugar canes, bananas, pineapples, cocoa, coffee, tobacco, ligneous plants, fruit or non-fruit trees, vines, ornamental plants.

5. Method according to Claim 4, in which said cereals are chosen from the group consisting of wheat, barley, rice, maize, spelt, oats, fonio, rye, sorghum and millet.

6. Method according to Claim 4, in which said ornamental plants are geranium.

7. Method according to any one of Claims 1 to 6, in which said mycorrhiza comprises an active strain of endomycorrhizal fungus.

8. Method according to Claims 7, in which said active strain of endomycorrhizal fungus is an endomycorrhizal fungus *Sclerocystis sp.*

9. Method according to any one of Claims 1 to 8, in which said yeast extract is obtained by plasmolysis or autolysis, or a combination of plasmolysis and autolysis of yeasts, in particular of the genus *Saccharomyces, Kluyveromyces, Candida or Torula,* preferentially S. *cerevisiae.*

10. Method according to Claim 9, in which said yeast is of the genus *Saccharomyces, Kluyveromyces, Candida* or *Torula.*

11. Method according to Claim 10, in which said yeast is *S*. *cerevisiae.*

12. Method according to any one of Claims 1 to 11, in which the yeast extract and the mycorrhiza are administered simultaneously or successively, by application to the soil.

13. Method according to Claim 12, in which said application to the soil is selected from the group consisting of spraying, spreading, sprinkling, fertigation, dropwise, in the seed drill or in the open field , by root dipping, by seed treatment or by incorporation into a cultivation support or by any means which makes it possible to bring the composition into contact, immediately or in the future, with the roots to be inoculated

14. Composition comprising at least one mycorrhiza and at least one yeast extract corresponding to the content of the yeast cells with the exclusion of the shells and obtained by a process selected from the group comprising plasmolysis, autolysis, and the combination of plasmolysis and autolysis, wherein the weight ratio of mycorrhizae to yeast extract is between 0.01 and 100.

15. Composition according to Claim 14, in which said mycorrhiza and said yeast extract are mixed in the same container or placed in two separate containers.

16. Composition according to Claim 14 or Claim 15, also comprising a substrate.

17. Composition according to any one of Claims 14 to 16, in which the amount of mycorrhizae is between 0.1 and 15 kg/m³ of the composition.

18. Composition according to Claim 17, in which the amount of mycorrhizae is between 0.5 and 8 kg/m³ of the composition.

19. Composition according to Claim 17, in which the amount of mycorrhizae is between 1 and 4 kg/m³ of the composition.

20. Composition according to any one of Claims 14 to 19, in which the amount of yeast extract is between 0.1 and 10 kg/m³ of the composition.

21. Composition according to Claim 20, in which the amount of yeast extract is between 0.2 and 5 kg/m³ of the composition

22. Composition according to Claim 20, in which the amount of yeast extract is between 0 .4 and 2 kg/m³ of the composition

23. Composition according to any one of Claims 14 to 22, in which the substrate comprises organic matter and inorganic matter.

24. Composition according to Claim 22, in which the organic matter is peat or the inorganic matter is selected from sand, clay.

25. Use of the composition according to one of Claims 14 to 24 for improving the growth and/or development and the productivity of non-leguminous plants.

26. Use according to Claim 25, **characterized in that** the provision of mycorrhiza is between 0.1 and 100 kg/ha.

27. Use according to Claim 25, **characterized in that** the provision of mycorrhiza is between 0.3 and 50 kg/ha.

28. Use according to Claim 25, **characterized in that** the provision of mycorrhiza is between 0.5 and 20 kg/ha.

29. Use according to any one of Claims 25 to 28, **characterized in that** the provision of yeast extract is between 0.1 and 50 kg/ha.

30. Use according to any one of Claims 25 to 28, **characterized in that** the provision of yeast extract is between 0.5 and 20 kg/ha.

31. Use according to any one of Claims 25 to 28, **characterized in that** the provision of yeast extract is between 1 and 10 kg/ha.

32. Process for producing a composition according to any one of Claims 14 to 24, comprising simultaneously or successively incorporating at least one mycorrhiza and at least one yeast extract corresponding to the content of the yeast cells with the exclusion of the shells and obtained by a process selected from the group comprising plasmolysis, autolysis, and the combination of plasmolysis and autolysis with a substrate.
